# EUROPÄISCHE PATENTSCHRIFT

(11) **EP 0 558 938 B1**
(45) Veröffentlichungstag und Bekanntmachung des Hinweises auf die Patenterteilung: **18.06.1997**
(21) Anmeldenummer: 93101544.0
(22) Anmeldetag: 02.02.1993
(51) Int. Cl.: C07C 61/15, C07C 55/32

(54) **Fluorsubstituierte Dicarbonsäuren sowie ein Verfahren zu ihrer Herstellung und Verwendung**
Fluoro-substituted dicarboxylic acids, process for preparing them and use
Acides dicarboxyliques substitués par fluor, leur procédé de préparation et leur emploi

(30) Priorität: 29.02.1992 DE 4206386
(43) Veröffentlichungstag der Anmeldung: 08.09.1993
(73) Patentinhaber: HOECHST AKTIENGESELLSCHAFT, 65926 Frankfurt am Main (DE)
(72) Erfinder: Kulpe, Jürgen, Dr., W-6230 Frankfurt/Main 80 (DE); Strutz, Heinz, Dr., W-6230 Frankfurt/Main 80 (DE)

(56) Entgegenhaltungen:
- EP-A- 0 352 718
- US-A- 2 453 146
- PATENT ABSTRACTS OF JAPAN, vol. 11, no. 394 (C-465)(2841), 23. Dezember 1987; & JP-A-62 153 241

## Beschreibung

Die vorliegende Erfindung betrifft fluorsubstituierte Dicarbonsäuren der allgemeinen Formel 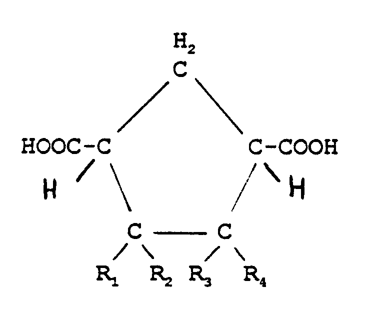 in der
R₁ H oder Fluor,
R₂ H oder Fluor,
R₃ H oder Fluor,
R₄ H, CF₃, (CF₂)₅CF₃, (CF₂)₇CF₃ oder (CH₂)₂-C₆F₁₃ bedeuten,
ein Verfahren zu ihrer Herstellung und ihre Verwendung.

In der JP-A 62 153 241 ist ein Verfahren zur Herstellung von fluorsubstituierten Dicarbonsäuren beschrieben, bei welche eine dihalogenierte Verbindung der Formel X-Rf-X', in der X, X' für Brom, Chlor oder Jod steht, mit CO₂ in einem aprotischen Lösungsmittel in Gegenwart von Zink umgesetzt wird und zu einer entsprechenden Dicarbonsäure führt. Rf steht dabei für eine gesättigte oder ungesättigte, geradkettig oder verzweigte, vollständig fluorierte Kette von 3 bis 20 C-Atomen.

Die EP 0 352 718-A1 beschreibt fluorsubstituierte Dicarbonsäureester, in denen die Carboxylgruppe jeweils über eine Ethylengruppe an den fluorsubstituierten Rest gebunden ist.

Fluorsubstituierte Dicarbonsäuren, welche einen cyclischen Kohlenstoffring enthalten, sind im vorgenannten Stand der Technik nicht enthalten.

Aus der großen Auswahl möglicher fluorsubstituierter Dicarbonsäuren sind folgende Verbindungen von besonderem Interesse:
a) 2,3,3-Trifluor-2-trifluormethylcyclopentan-1,4-dicarbonsäure 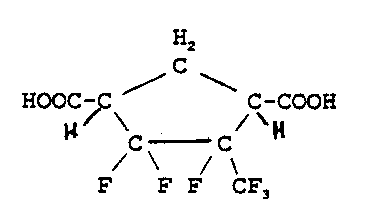
b) 2-Perfluorhexylcyclopentan-1,4-dicarbonsäure 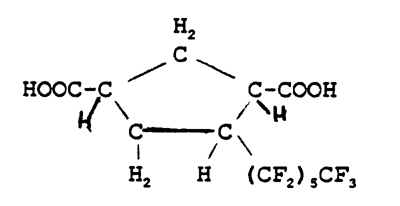
c) 2-Perfluoroctylcyclopentan-1,4-dicarbonsäure 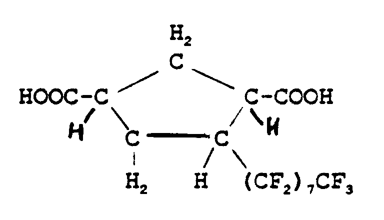
d) 2-(3,3,4,4,5,5,6,6,7,7,8,8,8)tridecafluoroctylcyclopentan-1,4-dicarbonsäure

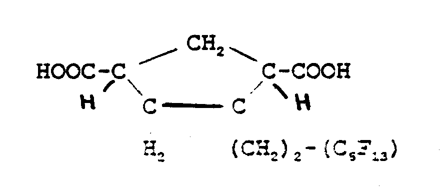

Als Ausgangspunkt für die Herstellung fluorsubstituierter Dicarbonsäuren wird auf das Umsetzungsprodukt von Butadien oder Cyclopentadien mit fluorsubstituiertem Alken zurückgegriffen. Die Umsetzung von Butadien bzw. Cyclopentadien mit Olefinen wird in der Literatur als "Diels-Alder-Reaktion" beschrieben.

Im J. Am. Chem. Soc. (1995), Vol. 77, Pag. 915 - 919 und J. Org. Chem. (1973), Vol. 38, No. 11, Pag. 2027 - 2042 sind Reaktionen zur Herstellung von Bicyclo[2.2.1]hepten-Derivaten beschrieben, welche Fluor enthalten und als Einsatzstoffe für die Herstellung der fluorsubstituierten Dicarbonsäuren geeignet sind.

Das Verfahren zur Herstellung der erfindungsgemäßen fluorsubstituierten Dicarbonsäuren aus dem Umsetzungsprodukt von Butadien oder Cyclopentadien mit fluorsubstituiertem Alken nach der Arbeitsweise einer Diels-Alder-Reaktion ist dadurch gekennzeichnet, daß man das Umsetzungsprodukt in einem Lösemittel löst und in diese Lösung äquimolare Mengen Ozon, bezogen auf das Umsetzungsprodukt, in Mischung mit Sauerstoff einleitet, das Lösemittel im Unterdruck abdestilliert, den erhaltenen Rückstand in einer Carbonsäure aufnimmt und diese Carbonsäurelösung mit Wasserstoffperoxid versetzt, durch Erwärmung miteinander zur Reaktion bringt und nach Abklingen der exothermen Reaktion weitere 0,5 bis 20 Stunden bei Temperaturen zwischen 50 und 120 °C nachreagieren läßt, anschließend die fluorsubstituierte Dicarbonsäure durch Abdestillieren der Carbonsäure und des Wassers gewinnt und gegebenenfalls in einem organischen Lösemittel durch eine Kristallisation reinigt.

Das Verfahren zur Herstellung der erfindungsgemäßen fluorsubstituierten Dicarbonsäuren kann wahlweise und bevorzugt dadurch gekennzeichnet sein, daß man
1) als Lösemittel für das Diels-Alder-Umsetzungsprodukt Ameisensäure, Essigsäure, Propionsäure, Methanol, Ethanol, Methylenchlorid, Tetrachlorkohlenstoff oder deren Gemische einsetzt,
2) das Ozon bei einer Temperatur zwischen -18 und 25 °C einleitet;
3) das ozonbehandelte Produkt in der 2- bis 1o-fachen Gewichtsmenge Ameisen-, Essig- oder Propionsäure als Carbonsäure aufnimmt;
4) beim Aufnehmen mit Essig- oder Propionsäure zusätzlich 0,1 bis 1,0 g konzentrierte Schwefelsäure pro 100 g Essig- oder Propionsäure zusetzt;
5) 3o bis 70 %iges Wasserstoffperoxid in einer Menge von 1 bis 5 Äquivalenten Oxidationsmittel pro Mol Diels-Alder-Umsetzungsprodukt einsetzt;
6) die Kristallisation der fluorsubstituierten Dicarbonsäure in Toluol mit einem Zusatz von 5 bis 2o Gewichtsprozenten Methyl-t-butylether oder in Methylenchlorid als organischem Lösemittel durchführt.

Die erfindungsgemäßen fluorsubstituierten Dicarbonsäuren können durch Polykondensation mit Diaminen zu ε-Lactam oder mit Diolen oder Polyolen zu Polyester umgesetzt werden. Diese ε-Lactame und Polyester zeichnen sich durch eine erhöhte thermische Beständigkeit aus.
Wahlweise kann die Verwendung fluorsubstituierter Dicarbonsäure zur Herstellung von ε-Lactam dadurch gekennzeichnet sein, daß man 5 bis 5o Molprozente der gesamten kondensierten Dicarbonsäure durch fluorsubstituierte Dicarbonsäure ersetzt.

Bei der gewählten Herstellungsart des Diels-Alder-Umsetzungsprodukts entsteht ein Isomerengemisch aus exo- und endo-Additionsprodukten. Es können aber auch die reinen exo- oder endo-Additionsprodukte einer Diels-Alder-Reaktion als Ausgangsprodukt für die Herstellung fluorsubstituierter Dicarbonsäuren eingesetzt werden.

### Beispiel A (Diels-Alder-Reaktion)

In einem 1 l Autoklav werden 554 ml = 864 g (2,5 mol) Perfluorhexylethen, 165 g (2,5 mol) frisch destilliertes Cyclopentadien und 5 g Hydrochinon vorgelegt. Der Autoklav wird zweimal mit Stickstoff gespült. Anschließend werden 5 bar Stickstoff aufgedrückt und der Autoklav auf 17o °C erwärmt. Nach 72 Stunden läßt man abkühlen, entspannt den Stickstoffüberdruck und filtriert den Autoklaveninhalt in eine Destillationsvorlage. Bei der fraktionierenden Vakuumdestillation werden 783 g des Diels-Alder-Produktes mit einem Siedepunkt von 33 - 35 °C bei einem Druck von 0,25 Torr isoliert. Durch ¹H-NMR-Spektroskopie wurde ein Endo/Exo-Isomerenverhältnis von 77/23 bestimmt.

Die nachfolgenden Beispiele sollen die Herstellung der erfindungsgemäßen Verbindungen erläutern:

### Beispiel 1

### Herstellung von 2,3,3-Trifluor-2-trifluormethylcyclopentan-1,4-dicarbonsäure

21,6 g (100 mmol) des Diels-Alder-Umsetzungsproduktes von Hexafluorpropen und Cyclopentadien werden in einem Gemisch aus 100 ml Essigsäure und 100 ml Ameisensäure gelöst und bei -5 °C eine Ozon-Sauerstoff-Mischung (40 bis 60 g O₃ pro Nm³O₂) durchgeleitet, bis 1 Äquivalent Ozon aufgenommen ist. In die Lösung werden 10,8 ml (250 mmol) 70 prozentiges Wasserstoffperoxid zugegeben und die Lösung auf 60 °C erwärmt. Durch die einsetzende exotherme Reaktion gerät die Lösung ins Sieden. Nach der Beendigung der exothermen Reaktion wird das Gemisch weitere 3 Stunden lang auf 8o °C gehalten. Eine Prüfung auf Oxidationsmittel mit KI-Stärkepapier ergab nach dieser Zeit keine Färbung. Das Lösemittel wird im Vakuum im Rotationsverdampfer bei Raumtemperatur abgezogen. Der ölige Rückstand (24 g) wird mit 3o ml Methylenchlorid versetzt und gerührt. Dabei fällt ein weißer kristalliner Niederschlag aus, der abgesaugt und getrocknet wird.
- Ausbeute:: 17,4 g = 62 % der Theorie.
- Schmelzpunkt:: 1o8-115 °C.

### Beispiel 2

### Herstellung von 3-Perfluorhexylcyclopentan-1,4-dicarbonsäure

2o,6 g (5o mmol) des Diels-Alder-Umsetzungsproduktes von 3,3,4,4,5,5,6,6,7,7,8,8,8-Tridecafluor-1-octen (Perfluorhexylethen) und Cyclopentadien werden in einem Gemisch aus 5o ml Methanol und 5o ml Methylenchlorid gelöst und bei -4o °C mit einer Ozon-Sauerstoff-Mischung (40 bis 60 g O₃ pro Nm³O₂) behandelt, bis 1 Äquivalent Ozon aufgenommen ist. Die Lösung nimmt eine blaue Farbe an. Bei Raumtemperatur werden im Vakuum die Leichtsieder abdestilliert. Der sirupöse Rückstand wird in 1oo ml Ameisensäure aufgenommen und bei 60 °C mit 7,5 ml (87,5 mmol) 35 prozentigem Wasserstoffperoxid versetzt. Durch die einsetzende exotherme Reaktion beginnt das Gemisch zu sieden. Nach Beendigung der exothermen Reaktion wird das Gemisch 2 Stunden lang auf 80 °C und danach 1 Stunde im Sieden gehalten. Der Rückstand wird aus Toluol/Methyl-t-butylether (10/1 Volumenteile) umkristallisiert.
- Ausbeute:: 15,5 g = 65 % der Theorie.
- Schmelzpunkt:: 123 - 137 °C.

### Beispiel 3

### Herstellung von 2-Perfluoroctylcyclopentan-1,4-dicarbonsäure

25,6 g (5o mmol) des Diels-Alder-Umsetzungsproduktes von 3,3,4,4,5,5,6,6,7,7,8,8,9,9,1o,1o,1o-Heptadecafluor-1-decen (Perfluoroctylethen) und Cyclopentadien werden in einem Gemisch aus 5o ml Methanol und 5o ml Methylenchlorid gelöst und bei 5 °C mit einer Ozon-Sauerstoff-Mischung (40 bis 60 g O₃pro Nm³O₂) behandelt, bis 1 Äquivalent Ozon aufgenommen ist. Bei Raumtemperatur werden die Lösemittel im Vakuum abdestilliert. Der sirupöse Rückstand wird in 1oo ml Ameisensäure aufgenommen und bei 6o °C mit 3,8 ml (87,5 mmol) 70 prozentigem Wasserstoffperoxid versetzt. Durch die einsetzende exotherme Reaktion beginnt das Gemisch zu sieden. Nach Beendigung der exothermen Reaktion wird das Gemisch weitere 2 Stunden lang auf 8o °C und eine weitere Stunde im Sieden gehalten. Nach Abkühlung auf Raumtemperatur wird der ausgefallene Niederschlag abgesaugt, getrocknet und aus Toluol/Methyl-t-butylether (1o/1 Volumenteile) umkristallisiert.
- Ausbeute:: 22 g = 76 % der Theorie.
- Schmelzpunkt:: 13o - 141 °C.

### Beispiel 4

### Herstellung von 2-(3,3,4,4,5,5,6,6,7,7,8,8,8-Tridecafluoroctyl)cyclopentan-1,4-dicarbonsäure

22,0 g (50 mmol) des Diels-Alder-Umsetzungsproduktes von 5,5,6,6,7,7,8,8,9,9,10,10,10-Tridecafluor-1-decen (Perfluorhexylbuten-1) und Cyclopentadien werden in 100 ml Methylenchlorid gelöst und bei 0 °C mit einer Ozon-Sauerstoff-Mischung (40 bis 60 g O₃ pro Nm O₂) behandelt, bis 1 Äquivalent Ozon aufgenommen ist. Bei Raumtemperatur werden im Vakuum die Leichtsieder abdestilliert. Der sirupöse Rückstand wird in 150 ml Ameisensäure aufgenommen und bei 60 °C mit 5,3 ml (88 mmol) 50%igem Wasserstoffperoxid versetzt. Durch die einsetzende exotherme Reaktion beginnt das Gemisch zu sieden. Nach Beendigung der exothermen Reaktion wird das Gemisch 20 Stunden lang auf 60 °C gehalten. Der ausgefallene Feststoff wird aus Toluol/Methyl-t-butylether (10/1 Volumenteile) umkristallisiert.
- Ausbeute:: 14,5 g = 29 % der Theorie

## Patentansprüche (Patentansprüche für folgende(n) Vertragsstaat(en): AT, BE, CH, DE, FR, GB, IT, LI, NL)

1. Fluorsubstituierte Carbonsäuren der allgemeinen Formel 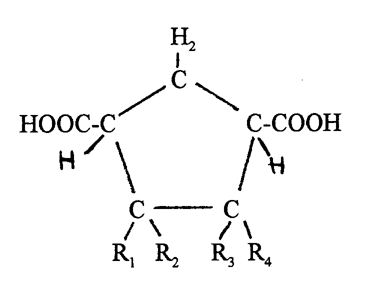 in der
R₁ H oder Fluor,
R₂ H oder Fluor,
R₃ H oder Fluor,
R₄ H, CF₃, (CF₂)₅CF₃, (CF₂)₇CF₃ oder (CH₂)₂ - C₆F₁₃
bedeuten.

2. Die fluorsubstituierte Dicarbonsäure nach Anspruch 1, dadurch gekennzeichnet, daß sie 2,3,3-Trifluor-2-trifluormethylcyclopentan-1,4-dicarbonsäure ist.

3. Die fluorsubstituierte Dicarbonsäure nach Anspruch 1, dadurch gekennzeichnet, daß sie 2-Perfluorhexylcyclopentan-1,4-dicarbonsäure ist.

4. Die fluorsubstituierte Dicarbonsäure nach Anspruch 1, dadurch gekennzeichnet, daß sie 2-Perfluoroctylcyclopentan-1,4-dicarbonsäure ist.

5. Die fluorsubstituierte Dicarbonsäure nach Anspruch 1, dadurch gekennzeichnet, daß sie 2-(3,3,4,4,5,5,6,6,7,7,8,8,8)-tridecafluoroctylcyclopentan-1,4-dicarbonsäure ist.

6. Verfahren zur Herstellung fluorsubstituierter Dicarbonsäuren, nach einem der Ansprüche 1 bis 5, aus dem Umsetzungsprodukt von Butadien oder Cyclopentadien mit fluorsubstituiertem Alken nach der Arbeitsweise einer Diels-Alder-Reaktion, dadurch gekennzeichnet, daß man das Umsetzungsprodukt in einem Lösemittel löst und in diese Lösung äquimolare Mengen Ozon, bezogen auf das Umsetzungsprodukt, in Mischung mit Sauerstoff einleitet, das Lösemittel im Unterdruck abdestilliert, den erhaltenen Rückstand in einer Carbonsäure aufnimmt und diese Carbonsäurelösung mit Wasserstoffperoxid versetzt, durch Erwärmung miteinander zur Reaktion bringt und nach Abklingen der exothermen Reaktion weitere 0,5 bis 20 Stunden bei Temperaturen zwischen 50 und 120 °C nachreagieren läßt, anschließend die fluorsubstituierte Dicarbonsäure durch Abdestillieren der Carbonsäure und des Wassers gewinnt und gegebenenfalls in einem organischen Lösemittel durch eine Kristallisation reinigt.

7. Verfahren nach Anspruch 7, dadurchgekennzeichnet, daß man als Lösemittel für das Diels-Alder-Umsetzungsprodukt Ameisensäure, Essigsäure, Propionsäure, Methanol, Ethanol, Methylenchlorid, Tetrachlorkohlenstoff oder deren Gemische einsetzt.

8. Verfahren nach Anspruch 6 oder 7, dadurch gekennzeichnet, daß man das Ozon bei einer Temperatur zwischen -18 und 25 °C einleitet.

9. Verfahren nach einem der Ansprüche 6 bis 8, dadurch gekennzeichnet, daß man das ozonbehandelte Produkt in der 2- bis 10-fachen Gewichtsmenge Ameisen-, Essig- oder Propionsäure als Carbonsäure aufnimmt.

10. Verfahren nach Anspruch 9, dadurch gekennzeichnet, daß man beim Aufnehmen mit Essig- oder Propionsäure zusätzlich 0,1 bis 1,0 g konzentrierte Schwefelsäure pro 100 g Essig- oder Propionsäure zusetzt.

11. Verfahren nach einem der Ansprüche 6 bis 10, dadurch gekennzeichnet, daß man 30 bis 70%iges Wasserstoffperoxid in einer Menge von 1 bis 5 Äquivalenten Oxidationsmittel pro Mol Diels-Alder-Umsetzungsprodukt einsetzt.

12. Verfahren nach einem der Ansprüche 6 bis 11, dadurch gekennzeichnet, daß man die Kristallisation der fluorsubstituierten Dicarbonsäure in Toluol mit einem Zusatz von 5 bis 20 Gewichtsprozenten Methyl-t-butylether oder in Methylenchlorid als organischem Lösemittel durchführt.

13. Verwendung fluorsubstituierter Dicarbonsäure nach einem der Ansprüche 1 bis 5 oder hergestellt nach einem der Ansprüche 6 bis 12 zur Herstellung von ε-Lactam oder Polyester.

14. Verwendung nach Anspruch 13 zur Herstellung von ε-Lactam, dadurch gekennzeichnet, daß man 5 bis 50 Molprozente der gesamten kondensierten Dicarbonsäure durch fluorsubstituierte Dicarbonsäure ersetzt.

15. Verwendung nach Anspruch 13 zur Herstellung von Polyester, dadurch gekennzeichnet, daß man 5 bis 50 Molprozente der gesamten kondensierten Dicarbonsäure durch fluorsubstituierte Dicarbonsäure ersetzt.

## Patentansprüche (Patentansprüche für folgende(n) Vertragsstaat(en): ES)

1. Verfahren zur Herstellung fluorsubstituierter Dicarbonsäuren der allgemeinen Formel 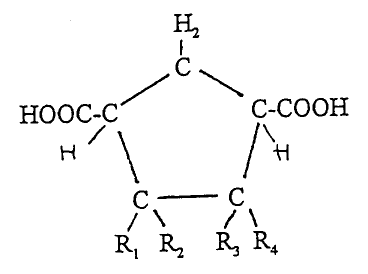 in der R₁ H oder Fluor, R₂ H oder Fluor, R₃ H oder Fluor, R₄ H, CF₃, (CF₂)₅CF₃, (CF₂)₇CF₃ oder (CH₂)₂-C₆F₁₃ bedeuten, aus dem Umsetzungsprodukt von Butadien oder Cyclopentadien mit fluorsubstituiertem Alkan nach der Arbeitsweise einer Diels-Alder-Reaktion, dadurch gekennzeichnet, daß man das Umsetzungsprodukt in einem Lösemittel löst und in diese Lösung äquimolare Mengen Ozon, bezogen auf das Umsetzungsprodukt, in Mischung mit Sauerstoff einleitet, das Lösemittel im Unterdruck abdestilliert, den erhaltenen Rückstand in einer Carbonsäure aufnimmt und diese Carbonsäurelösung mit Wasserstoffperoxid versetzt, durch Erwärmung miteinander zur Reaktion bringt und nach Abklingen der exothermen Reaktion weitere 0,5 bis 20 Stunden bei Temperaturen zwischen 50 und 120°C nachreagieren läßt, anschließend die fluorsubstituierte Dicarbonsäure durch Abdestillieren der Carbonsäure und des Wassers gewinnt und gegebenenfalls in einem organischen Lösemittel durch eine Kristallisation reinigt.

2. Verfahren nach Anspruch 1, dadurch gekennzeichnet, daß man als Lösemittel für das Diels-Alder-Umsetzungsprodukt Ameisensäure, Essigsäure, Propionsäure, Methanol, Ethanol, Methylenchlorid, Tetrachlorkohlenstoff oder deren Gemische einsetzt.

3. Verfahren nach Anspruch 1 oder 2, dadurch gekennzeichnet, daß man das Ozon bei einer Temperatur zwischen -18 und 25°C einleitet.

4. Verfahren nach einem der Ansprüche 1 bis 3, dadurch gekennzeichnet, daß man das ozonbehandelte Produkt in der 2- bis 10-fachen Gewichtsmenge Ameisen-, Essig- oder Propionsäure als Carbonsäure aufnimmt.

5. Verfahren nach Anspruch 4, dadurch gekennzeichnet, daß man beim Aufnehmen mit Essig- oder Propionsäure zusätzlich 0,1 bis 1,0 g konzentrierte Schwefelsäure pro 100 g Essig- oder Propionsäure zusetzt.

6. Verfahren nach einem der Ansprüche 1 bis 5, dadurch gekennzeichnet, daß man 30 bis 70 %iges Wasserstoffperoxid in einer Menge von 1 bis 5 Äquivalenten Oxidationsmittel pro Mol Diels-Alder-Umsetzungsprodukt einsetzt.

7. Verfahren nach einem der Ansprüche 1 bis 6, dadurch gekennzeichnet, daß man die Kristallisation der fluorsubstituierten Dicarbonsäure in Toluol mit einem Zusatz von 5 bis 20 Gewichtsprozenten Methyl-t-butylether oder in Methylenchlorid als organischem Lösemittel durchführt.

8. Verwendung fluorsubstituierter Dicarbonsäure hergestellt nach einem der Ansprüche 1 bis 6 zur Herstellung von ε-Lactam oder Polyester.

9. Verwendung nach Anspruch 8 zur Herstellung von ε-Lactam, dadurch gekennzeichnet, daß man 5 bis 50 Molprozente der gesamten kondensierten Dicarbonsäure durch fluorsubstituierte Dicarbonsäure ersetzt.

10. Verwendung nach Anspruch 8 zur Herstellung von Polyester, dadurch gekennzeichnet, daß man 5 bis 50 Molprozente der gesamten kondensierten Dicarbonsäure durch fluorsubstituierte Dicarbonsäure ersetzt.

## Claims (Claims for the following Contracting State(s): AT, BE, CH, DE, FR, GB, IT, LI, NL)

1. A fluorine-substituted dicarboxylic acid of the formula 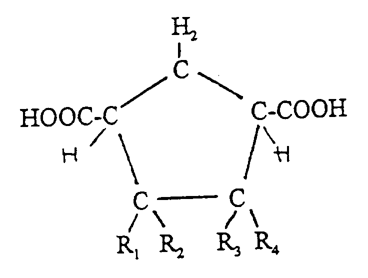 in which
R₁ is H or fluorine,
R₂ is H or fluorine,
R₃ is H or fluorine and
R₄ is H, CF₃, (CF₂)₅CF₃, (CF₂)₇CF₃ or (CH₂)₂-C₆F₁₃.

2. A fluorine-substituted dicarboxylic acid as claimed in claim 1, which is 2,3,3-trifluoro-2-trifluoromethylcyclopentane-1,4-dicarboxylic acid.

3. A fluorine-substituted dicarboxylic acid as claimed in claim 1, which is 2-perfluorohexylcyclopentane-1,4dicarboxylic acid.

4. A fluorine-substituted dicarboxylic acid as claimed in claim 1, which is 2-perfluorooctylcyclopentane-1,4-dicarboxylic acid.

5. A fluorine-substituted dicarboxylic acid as claimed in claim 1, which is 2-(3,3,4,4,5,5,6,6,7,7,8,8,8)-tridecafluorooctylcyclopentane-1,4-dicarboxylic acid.

6. A process for the preparation of fluorine-substituted dicarboxylic acids as claimed in any of claims 1 to 5, from the product of the reaction of butadiene or cyclopentadiene with fluorine-substituted alkene by means of a Diels-Alder reaction, which comprises the reaction product being dissolved in a solvent and an equimolar amount of ozone, based on the amount of the reaction product, mixed with oxygen being passed into this solution, the solvent being distilled off under reduced pressure, the resulting residue being taken up in a carboxylic acid and this carboxylic acid solution being mixed with hydrogen peroxide, reaction together being brought about by heating, and after the exothermic reaction has subsided the reaction being continued for a further 0.5 to 20 hours at temperatures between 50 and 120°C, and subsequently the fluorine-substituted dicarboxylic acid being isolated by distilling off the carboxylic acid and water and, if necessary, purified by crystallization from an organic solvent.

7. The process as claimed in claim 6, wherein formic acid, acetic acid, propionic acid, methanol, ethanol, methylene chloride, carbon tetrachloride or a mixture thereof is used as solvent for the Diels-Alder reaction product.

8. The process as claimed in claim 6 or 7, wherein the ozone is passed in at a temperature between -18 and 25°C.

9. The process as claimed in any of claims 6 to 8, wherein the ozone-treated product is taken up in 2 to 10 times the amount by weight of formic, acetic or propionic acid as carboxylic acid.

10. The process as claimed in claim 9, wherein an additional 0.1 to 1.0 g of concentrated sulfuric acid per 100 g of acetic or propionic acid is added when taking up in acetic or propionic acid.

11. The process as claimed in any of claims 6 to 10, wherein 30 to 70% strength hydrogen peroxide is added in an amount of 1 to 5 equivalents of oxidizing agent per mole of Diels-Alder reaction product.

12. The process as claimed in any of claims 6 to 11, wherein the crystallization of the fluorine-substituted dicarboxylic acid is carried out in toluene, with the addition of 5 to 20 percent by weight of methyl t-butyl ether, or in methylene chloride as organic solvent.

13. The use of a fluorine-substituted dicarboxylic acid as claimed in any of claims 1 to 5 of prepared as claimed in any of claims 6 to 12 for the preparation of ε-lactam or polyester.

14. The use as claimed in claim 13 for the preparation of ε-lactam, wherein 5 to 50 mol percent of the total condensed dicarboxylic acid are replaced by fluorine-substituted dicarboxylic acid.

15. The use as claimed in claim 13 for the preparation of polyester, wherein 5 to 50 mol percent of the total condensed dicarboxylic acid are replaced by fluorine-substituted dicarboxylic acid.

## Claims (Claims for the following Contracting State(s): ES)

1. A process for the preparation of a fluorine-substituted dicarboxylic acid of the formula 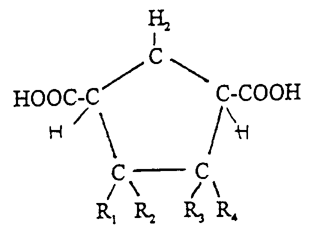 in which R₁ is H or fluorine, R₂ is H or fluorine, R₃ is H or fluorine and R₄ is H, CF₃, (CF₂)₅CF₃, (CF₂)₇CF₃ or (CH₂)₂-C₆F₁₃, from the product of the reaction of butadiene or cyclopentadiene with fluorine-substituted alkene by means of a Diels-Alder reaction, which comprises the reaction product being dissolved in a solvent and an equimolar amount of ozone, based on the amount of the reaction product, mixed with oxygen being passed into this solution, the solvent being distilled off under reduced pressure, the resulting residue being taken up in a carboxylic acid and this carboxylic acid solution being mixed with hydrogen peroxide, reaction together being brought about by heating, and after the exothermic reaction has subsided the reaction being continued for a further 0.5 to 20 hours at temperatures between 50 and 120°C, and subsequently the fluorine-substituted dicarboxylic acid being isolated by distilling off the carboxylic acid and water and, if necessary, purified by crystallization from an organic solvent.

2. The process as claimed in claim 1, wherein formic acid, acetic acid, propionic acid, methanol, ethanol, methylene chloride, carbon tetrachloride or a mixture thereof is used as solvent for the Diels-Alder reaction product.

3. The process as claimed in claim 1 or 2, wherein the ozone is passed in at a temperature between -18 and 25°C.

4. The process as claimed in any of claims 1 to 3, wherein the ozone-treated product is taken up in 2 to 10 times the amount by weight of formic, acetic or propionic acid as carboxylic acid.

5. The process as claimed in claim 4, wherein an additional 0.1 to 1.0 g of concentrated sulfuric acid per 100 g of acetic or propionic acid is added when taking up in acetic or propionic acid.

6. The process as claimed in any of claims 1 to 5, wherein 30 to 70% strength hydrogen peroxide is added in an amount of 1 to 5 equivalents of oxidizing agent per mole of Diels-Alder reaction product.

7. The process as claimed in any of claims 1 to 6, wherein the crystallization of the fluorine-substituted dicarboxylic acid is carried out in toluene, with the addition of 5 to 20 percent by weight of methyl t-butyl ether, or in methylene chloride as organic solvent.

8. The use of a fluorine-substituted dicarboxylic acid prepared as claimed in any of claims 1 to 6 for the preparation of ε-lactam or polyester.

9. The use as claimed in claim 8 for the preparation of ε-lactam, wherein 5 to 50 mol percent of the total condensed dicarboxylic acid are replaced by fluorine-substituted dicarboxylic acid.

10. The use as claimed in claim 8 for the preparation of polyester, wherein 5 to 50 mol percent of the total condensed dicarboxylic acid are replaced by fluorine-substituted dicarboxylic acid.

## Revendications (Revendications pour l'(les) Etat(s) contractant(s) suivant(s): AT, BE, CH, DE, FR, GB, IT, LI, NL)

1. Acides carboxyliques substitués par fluor, représentés par la formule générale : 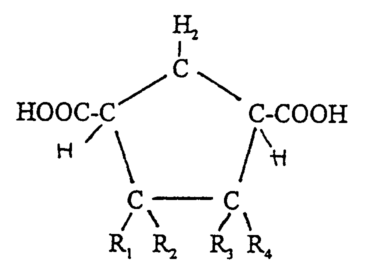 dans laquelle :
- R₁ représente H ou fluor ;
- R₂ représente H ou fluor ;
- R₃ représente H ou fluor ;
- R₄ représente H, CF₃, (CF₂)₅CF₃, (CF₂)₇CF₃ ou (CH₂)₂-C₆F₁₃.

2. Acide dicarboxylique substitué par fluor selon la revendication 1, caractérisé par le fait qu'il est l'acide 2,3,3-trifluoro-2-trifluorométhylcyclopentane-1,4-dicarboxylique.

3. Acide dicarboxylique substitué par fluor selon la revendication 1, caractérisé par le fait qu'il est l'acide 2-perfluorohexylcyclopentane-1,4-dicarboxylique.

4. Acide dicarboxylique substitué par fluor selon la revendication 1, caractérisé par le fait qu'il est l'acide 2-perfluorooctylcyclopentane-1,4-dicarboxylique.

5. Acide dicarboxylique substitué par fluor selon la revendication 1, caractérisé par le fait qu'il est l'acide 2-(3,3,4,4,5,5,6,6,7,7,8,8,8)-tridécafluorooctylcyclopentane-1,4-dicarboxylique.

6. Procédé de fabrication d'acides dicarboxyliques substitués par fluor, tels que définis à l'une des revendications 1 à 5, à partir du produit de réaction du butadiène ou du cyclopentadiène avec un alcène substitué par fluor d'après la méthode d'une réaction de Diels-Alder, caractérisé par le fait qu'on dissout le produit de réaction dans un solvant et qu'on introduit, dans cette solution, des quantités équimolaires d'ozone, par rapport au produit de réaction, en mélange avec de l'oxygène, qu'on élimine le solvant par distillation sous pression réduite, qu'on reprend le résidu obtenu dans un acide carboxylique et qu'on mélange cette solution d'acide carboxylique avec du peroxyde d'hydrogène, qu'on les amène à réagir ensemble par chauffage et qu'après décroissance de la réaction exothermique, on laisse la réaction se poursuivre pendant encore 0,5 à 20 heures à des températures comprises entre 50 et 120°C, qu'ensuite on obtient l'acide dicarboxylique substitué par fluor par élimination par distillation de l'acide carboxylique et de l'eau et, le cas échéant, on le purifie dans un solvant organique par une cristallisation.

7. Procédé selon la revendication 6, caractérisé par le fait qu'on utilise, comme solvant pour le produit de réaction de Diels-Alder, l'acide formique, l'acide acétique, l'acide propionique, le méthanol, l'éthanol, le chlorure de méthylène, le tétrachlorure de carbone ou leurs mélanges.

8. Procédé selon l'une des revendications 6 ou 7, caractérisé par le fait qu'on introduit l'ozone à une température comprise entre -18 et 25°C.

9. Procédé selon l'une des revendications 6 à 8, caractérisé par le fait qu'on reprend le produit traité par l'ozone dans la quantité pondérale de 2 à 10 fois d'acide formique, acétique ou propionique comme acide carboxylique.

10. Procédé selon la revendication 9, caractérisé par le fait qu'on ajoute, lors de la reprise par l'acide acétique ou propionique, encore 0,1 à 1,0 g d'acide sulfurique concentré pour 100 g d'acide acétique ou propionique.

11. Procédé selon l'une des revendications 6 à 10, caractérisé par le fait qu'on utilise du peroxyde d'hydrogène à 30 à 70% dans une quantité de 1 à 5 équivalents d'agent d'oxydation par mole de produit de réaction de Diels-Alder.

12. Procédé selon l'une des revendications 6 à 11, caractérisé par le fait qu'on conduit la cristallisation de l'acide dicarboxylique substitué par fluor dans du toluène avec une addition de 5 à 20 pour cent en poids de méthyl t-butyl éther ou dans du chlorure de méthylène comme solvant organique.

13. Utilisation de l'acide dicarboxylique substitué par fluor tel que défini à l'une des revendications 1 à 5 ou préparé par un procédé tel que défini à l'une des revendications 6 à 12 pour la fabrication d'ε-lactames ou de polyesters.

14. Utilisation selon la revendication 13, pour la fabrication d'ε-lactames, caractérisée par le fait qu'on remplace 5 à 50 pour cent en moles de la totalité de l'acide dicarboxylique condensé par l'acide dicarboxylique substitué par fluor.

15. Utilisation selon la revendication 13 pour la fabrication de polyesters, caractérisée par le fait qu'on remplace 5 à 50 pour cent en moles de la totalité de l'acide dicarboxylique condensé par l'acide dicarboxylique substitué par fluor.

## Revendications (Revendications pour l'(les) Etat(s) contractant(s) suivant(s): ES)

1. Procédé de fabrication d'acides dicarboxyliques substitués par fluor, représentés par la formule générale : 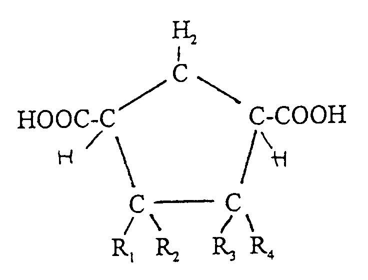 dans laquelle :
- R₁ représente H ou fluor ;
- R₂ représente H ou fluor ;
- R₃ représente H ou fluor ;
- R₄ représente H, CF₃, (CF₂)₅CF₃, (CF₂)₇CF₃ ou (CH₂)₂-C₆F₁₃,
à partir du produit de réaction du butadiène ou du cyclopentadiène avec un alcane substitué par fluor d'après la méthode d'une réaction de Diels-Alder, caractérisé par le fait qu'on dissout le produit de réaction dans un solvant et qu'on introduit, dans cette solution, des quantités équimolaires d'ozone, par rapport au produit de réaction, en mélange avec de l'oxygène, qu'on élimine le solvant par distillation sous pression réduite, qu'on reprend le résidu obtenu dans un acide carboxylique et qu'on mélange cette solution d'acide carboxylique avec du peroxyde d'hydrogène, qu'on les amène à réagir ensemble par chauffage et qu'après décroissance de la réaction exothermique, on laisse la réaction se poursuivre pendant encore 0,5 à 20 heures à des températures comprises entre 50 et 120°C, qu'ensuite on obtient l'acide dicarboxylique substitué par fluor par élimination par distillation de l'acide carboxylique et de l'eau et le cas échéant qu'on le purifie dans un solvant organique par une cristallisation.

2. Procédé selon la revendication 1, caractérisé par le fait qu'on utilise, comme solvant pour le produit de réaction de Diels-Alder, l'acide formique, l'acide acétique, l'acide propionique, le méthanol, l'éthanol, le chlorure de méthylène, le tétrachlorure de carbone ou leurs mélanges.

3. Procédé selon l'une des revendications 1 ou 2, caractérisé par le fait qu'on introduit l'ozone à une température comprise entre -18 et 25°C.

4. Procédé selon l'une des revendications 1 à 3, caractérisé par le fait qu'on reprend le produit traité par l'ozone dans la quantité pondérale de 2 à 10 fois d'acide formique, acétique ou propionique comme acide carboxylique.

5. Procédé selon la revendication 4, caractérisé par le fait qu'on ajoute, lors de la reprise par l'acide acétique ou propionique, encore 0,1 à 1,0 g d'acide sulfurique concentré pour 100 g d'acide acétique ou propionique.

6. Procédé selon l'une des revendications 1 à 5, caractérisé par le fait qu'on utilise du peroxyde d'hydrogène à 30 à 70% dans une quantité de 1 à 5 équivalents d'agent d'oxydation par mole de produit de réaction de Diels-Alder.

7. Procédé selon l'une des revendications 1 à 6, caractérisé par le fait qu'on conduit la cristallisation de l'acide dicarboxylique substitué par fluor dans du toluène avec une addition de 5 à 20 pour cent en poids de méthyl t-butyl éther ou dans du chlorure de méthylène comme solvant organique.

8. Utilisation d'un acide dicarboxylique substitué par fluor préparé par le procédé tel que défini à l'une des revendications 1 à 6 pour la fabrication d'ε-lactames ou de polyesters.

9. Utilisation selon la revendication 8, pour la fabrication d'ε-lactames, caractérisée par le fait qu'on remplace 5 à 50 pour cent en moles de la totalité de l'acide dicarboxylique condensé par l'acide dicarboxylique substitué par fluor.

10. Utilisation selon la revendication 8, pour la fabrication de polyesters, caractérisée par le fait qu'on remplace 5 à 50 pour cent en moles de la totalité de l'acide dicarboxylique condensé par l'acide dicarboxylique substitué par fluor.
